# EUROPÄISCHE PATENTANMELDUNG

(11) **EP 4 703 038 A1**
(43) Veröffentlichungstag der Anmeldung: **04.03.2026**
(21) Anmeldenummer: 24197639.8
(22) Anmeldetag: 30.08.2024
(51) Int. Cl.: B01L 3/00, B01L 9/00

(54) **BELEUCHTUNGSMODUL FÜR EINE MULTIWELL-PLATTE**

(71) Anmelder: OptoLumina AG, 3012 Bern (CH)
(72) Erfinder: HÖHENER, Thomas, 3012 Bern (CH)
(74) Vertreter: Clerc, Natalia

(57) **Zusammenfassung**

Ein Beleuchtungsmodul für eine Multiwell-Platte (2) weist ein Gehäuse (1,3), eine Beleuchtungseinheit (5) und eine Positionierungseinheit (15, 14) zur Positionierung der Multiwell-Platte (2) im Beleuchtungsmodul auf. Die Positionierungseinheit (15, 14) weist Lichtkanäle (16) auf, die voneinander getrennte Lichtverbindungen von der Beleuchtungseinheit (5) zu einer Oberfläche (151) der Positioniereinheit (15, 14) schaffen. Die Positionierungseinheit (15, 14) bildet an dieser Oberfläche (151) eine Auflagefläche zur abstandlosen Auflage eines Bodens (23) der Multiwell-Platte (2), der eine Unterseite der zellkulturaufnehmenden Vertiefungen (22) der Multiwell-Platte (2) bildet. Die abstandlose Aufnahme ist unabhängig von der Wellbodenhöhe, d.h. der "well bottom elevation", der Multiwell-Platte (2). Das erfindungsgemässe Beleuchtungsmodul ermöglicht die Verwendung von Multiwell-Platten unterschiedlicher Hersteller und unterschiedlicher Typen und Marken.

## Beschreibung

### TECHNISCHES GEBIET

Die vorliegende Erfindung betrifft ein Beleuchtungsmodul für eine Multiwell-Platte, eine Beleuchtungsvorrichtung mit einem Beleuchtungsmodul. Multiwell-Platten werden auch Wellplatten oder Zellkulturtestplatten genannt.

### STAND DER TECHNIK

Beleuchtungsvorrichtungen bzw. Beleuchtungsgeräte für Multiwell-Platten (in Deutsch auch Mikrotiterpatte und in Englisch Wellplates oder Multiwellplates) werden in der Pflanzen-, Photo- und zunehmend auch in der optogenetischen Biologie verwendet. Multiwell-Platten sind Platten, die eine Vielzahl an gleich grossen Vertiefungen, auch Kavitäten oder Wells genannt, zur Aufnahme von Proben, insbesondere von Zellkulturproben aufweisen. Multiwell-Platten weisen üblicherweise 6, 24 oder 96 und seltener 12, 48, 384, 1536 und 3456 Vertiefungen auf. Jede Vertiefung fungiert als kleines Reagenzglas und ermöglicht somit ein paralleles Testen von vielen Bedingungen mit einer einzigen Multiwell-Platte. Multiwell-Platte sind üblicherweise aus Kunststoff oder Glas gefertigt. Sie sind üblicherweise für sichtbares Licht transparent.

Optogenetik ist eine neuartige biologische Technologie mit grossem Potential. Optogenetik führt nicht nur zur Steigerung der Produktionsausbeute in der Biotechnologie, sondern liefert auch bei Wirkstoff-screens und anderen Forschungsanwendungen interessante Ergebnisse. Einsatzbereiche liegen nicht nur in der akademischen Forschung, sondern beispielsweise auch in der Pharmaindustrie, in der Medizinaltechnik und der Biotechnology. Weitere Gebiete, in welchen Beleuchtungsgeräte zum Einsatz kommen, umfassen unter anderem die Photopharmakologie, die optischen Chemie oder die Botanik.

In der Optogenetik nutzt man künstliche lichtsensitive Proteine, beispielsweise Enzyme oder Rezeptoren anstelle von Chemikalien um Funktionen von Zellen zu aktivieren und/oder zu deaktivieren und sie so zu manipulieren. Die Optogenetik ermöglicht eine hohe Präzision, da einzelne Zellen individuell aktiviert werden können. Die zeitliche Kontrolle ist gewährleistet, da einzelne Zellen nach Belieben ein- und ausgeschaltet werden können. Zudem lässt sich für praktisch jeden Prozess der Zelle ein optogenetisches Tool erstellen. Um die Prozesse zu aktivieren, sind jedoch spezifische Lichtquellen notwendig und Störungen durch fremde Lichtquellen müssen vermieden werden.

Effiziente Forschung und Entwicklung in den genannten Bereichen erfordern eine hohe Durchsatzrate an Proben und die Möglichkeit zur Parallelisierung von Experimenten. Dies lässt sich mit Pipettierrobotern und Multiwell-Platten erreichen.

Da in der Optogenetik die einzelnen Vertiefungen individuell und gezielt beleuchtet werden müssen, muss jede Vertiefung der Multiwell-Platte individuell und ohne Streulicht von benachbarten Lichtquellen oder Vertiefungen beleuchtet werden. Temperaturschwankungen sind ebenfalls zu vermeiden. Entsprechend hohe Anforderungen werden an die Beleuchtungsgeräte gestellt.

Ein Beleuchtungsgerät für Multiwell-Platten ist beispielsweise aus WO 03/051669 A1 bekannt.

Thomas Christoph Höhener et al, LITOS: a versatile LED illumination tool for optogenetic stimulation, Scientific Reports (2022) 12:13139, beschreibt ein Beleuchtungsgerät, das kostengünstig ist und einfach zusammengesetzt werden kann. Lichtstimulierungs-Programme lassen sich einfach erstellen und das Gerät lässt sich mit unterschiedlichen Wellplatten, Petrischalen, Zellkulturröhrchen und Zellkulturflaschen verwenden. Es ist für den Einsatz in Labors mit geringen finanziellen Mitteln geeignet.

CN 213951220 U zeigt ein Beleuchtungsgerät für Optogenetik mit einem Basisgehäuse, einer in das Basisgehäuse eingeschobenen LED-Array-Platte, einer über der LED-Array-Platte angeordneten Distanzplatte mit Lichtkanälen und mit einer Multiwell-Platte mit Vertiefungen, die jeweils genau über einem Lichtkanal zu liegen kommen. Die Distanzplatte weist hierzu eine rechteckige Ausnehmung auf, die die Multiwell-Platte aufnimmt.

Auch Lukasz J. Bugaj et al, High-thoughput multicolor optogenetics in microwell plates, Nature Protocols, Vo 14, July 2019, 2205-2228, verwendet eine Distanzplatte mit Lichtkanälen als Adaptor zwischen einer LED-Array-Platte und der Multiwell-Platte.

US 2018/0016538 A1 verwendet mehrere Adapter-Lagen. Zwischen den einzelnen Lagen ist ein Diffusorpapier für jede Vertiefung vorhanden zur Diffusion des LED Lichts.

Die Dimensionen der Multiwell-Platten und bei einigen Formaten auch die Position der Wells unterliegen Normen. Trotzdem gibt es grosse Unterschiede in der Gestaltung der Multiwell-Platten. Insbesondere die sogenannte "well bottom elevation", d.h. die Wellbodenhöhe, auch Kavitätbodenhöhe genannt, ist nicht normiert.

Die "well bottom elevation" ist der Abstand zwischen dem untersten Punkt der Innenfläche der Vertiefung und einer Auflagefläche, auf der die Multiwell-Platte aufliegt. Dieser Abstand wird vor Allem durch die konkrete Gestaltung eines umlaufenden und nach unten vorstehenden Randes der Multiwell-Platte definiert. Die Materialdicke der Multiwell-Platte, welche die Innenseite der Vertiefung von der Aussenseite trennt, trägt ebenfalls zur "well bottom elevation" bei, jedoch üblicherweise in einem geringeren Mass.

Diese "well bottom elevation", insbesondere der Abstand bis zur Aussenseite des Bodens der Vertiefung, beeinflusst die Qualität der Beleuchtung massgeblich. Stimmt der Abstand nicht, so ist eine gleichmässige Beleuchtung einer Vertiefung, d.h. eines Wells, ohne Beleuchtung der benachbarten Wells kaum möglich. Ausserdem ändert sich mit unterschiedlichem Abstand die Beleuchtungstärke, d.h. die Power Density, gemessen in mW/mm², was die Reproduzierbarkeit zwischen verschiedenen Multiwell-Platten beeinträchtigt.

Deshalb benötigt jeder Typ Multiwell-Platten ein eigenes Beleuchtungsgerät, um eine optimale Beleuchtung zu gewährleisten. D.h. Beleuchtungsgeräte müssen üblicherweise an einen Typ Multiwell-Platten von einem Hersteller angepasst werden. Somit beleuchtet ein Beleuchtungsgerät nur einen Typ Wellplate optimal, dies behindert den Wettbewerb, schränkt die Flexibilität ein und kann zu Versorgungsschwierigkeiten führen, da nicht einfach der Hersteller der Wellplatten gewechselt werden kann.

### DARSTELLUNG DER ERFINDUNG

Es ist deshalb eine Aufgabe der Erfindung, ein Beleuchtungsmodul für eine Multiwell-Platte zu schaffen, das eine grössere Flexibilität in der Wahl der Multiwell-Platten ermöglicht.

Diese Aufgabe löst ein Beleuchtungsmodul mit den Merkmalen des Patentanspruchs 1.

Das erfindungsgemässe Beleuchtungsmodul für eine Multiwell-Platte weist ein Gehäuse, eine Beleuchtungseinheit, eine Positionierungseinheit zur Positionierung der Multiwell-Platte im Beleuchtungsmodul und Lichtkanäle auf. Die Lichtkanäle schaffen voneinander getrennte Lichtverbindungen von der Beleuchtungseinheit zu einer Oberfläche der Positioniereinheit. Die Positionierungseinheit bildet an dieser Oberfläche eine Auflagefläche aus zur abstandlosen Auflage eines Bodens der Multiwell-Platte, der eine Unterseite der zellkulturaufnehmenden Vertiefungen der Multiwell-Platten bildet. Die abstandlose Aufnahme ist dabei unabhängig von einer Wellbodenhöhe, d.h. einer "well bottom elevation", der Multiwell-Platte.

Das erfindungsgemässe Beleuchtungsmodul ermöglicht einen stets gleichbleibenden Abstand zwischen der Unterseite der Vertiefungen, unabhängig von der "well bottom elevation", d.h. von der Wellbodenhöhe. Multiwell-Platten unterschiedlicher Typen und Marken, insbesondere von unterschiedlichen Herstellern, lassen sich somit mit gleichbleibender Qualität des Ergebnisses in demselben Beleuchtungsmodul verwenden.

Da sich die Lichtkanäle bis zu dieser Auflagefläche erstrecken, ist kein Spalt vorhanden zwischen den Wellböden, d.h. der unteren äusseren Oberfläche der Vertiefungen der Multiwell-Platte, und der Fläche, auf der die Multiwell-Platte aufsteht. Der Abstand von der mindestens einen Lichtquelle der Beleuchtungseinheit und den zellkulturaufnehmenden Vertiefungen ist stets gleich gross. Die Beleuchtungsstärke ist somit gleichbleibend und muss nicht durch eine Steuereinheit angepasst werden. Dies trifft sogar bei Verwendung von Wellplatten verschiedener Hersteller zu.

Das Beleuchtungsmodul ist somit universeller einsetzbar. Zudem kann die Steuerung einer zugehörigen Beleuchtungsvorrichtung vereinfacht werden, was die Entwicklungs- und Herstellungskosten eines Beleuchtungsgeräts reduziert.

Zudem wird verhindert, dass benachbarte Wells, d.h. Vertiefungen, unbeabsichtigt mit der falschen Lichtquelle beleuchtet werden.

Die abstandlose Auflage lässt sich auf unterschiedliche Weise erzielen. Beispielsweise ist die Auflagefläche manuell oder motorisch höhenverstellbar ausgebildet. Alternativ oder zusätzlich kann die Positionierungseinheit Einlagescheiben aufweisen, um die Auflagefläche zu erhöhen. Diese Einlagescheiben weisen vorzugsweise ebenfalls voneinander getrennte Lichtkanäle auf, die mit den Lichtkanälen einer Oberfläche des Beleuchtungsmoduls fluchten. Die Einlagescheiben lassen sich vorzugsweise auf die Oberfläche des Beleuchtungsmoduls auflegen, wodurch die Multiwell-Platte auf der obersten Einlageschale abstandlos aufliegt.

In bevorzugten Ausführungsformen weist die Positioniereinheit einen Trägerblock und eine den Trägerblock umlaufende Nut auf, wobei eine oberste Oberfläche des Trägerblocks die Auflagefläche bildet.

In einigen Ausführungsformen ist der Trägerblock höhenverstellbar und/oder kann mit den oben beschriebenen Einlagescheiben belegt werden. Vorzugsweise ist er jedoch ortsfest und nicht verstellbar im Gehäuse des Beleuchtungsmoduls angeordnet.

In einigen Ausführungsformen ist der Trägerblock austauschbar im Gehäuse des Beleuchtungsmoduls angeordnet. Dadurch lässt sich dasselbe Beleuchtungsmodul mit verschiedenen Trägerblöcken verwenden, die beispielsweise eine unterschiedliche Anzahl von Lichtkanälen aufweisen und somit für Multiwell-Platten mit der entsprechenden Anzahl von Wells verwendet werden können.

In anderen Ausführungsformen ist der Trägerblock fest mit dem Gehäuse verbunden oder er ist ein Bestandteil des Gehäuses. Vorzugsweise ist er monolithisch mit mindestens einem Teil des Gehäuses ausgebildet.

Der Trägerblock und/oder das Gehäuse bestehen vorzugsweise aus Aluminium.

Vorzugsweise weist die Nut eine Tiefe auf, die mindestens 3.5 mm, vorzugsweise mindestens 5 mm beträgt. Dies ist grösser als die Höhe der Wellboden, d.h. die "well bottom elevation" handelsüblicher Multiwell-Platten. Dadurch ist sichergestellt, dass handelsüblichen Multiwell-Platten nicht mit ihren nach unten vorstehenden umlaufenden Rändern auf dem Grund der Nut aufliegen und somit ein Abstand zwischen der Auflagefläche und den Wellböden entsteht.

Die äussere Kante der Nut weist vorzugsweise eine Länge von 128.25 +0.2/-0.0 mm und eine Breite von 86 +0.2/-0.0 mm auf. Dies ist leicht grösser als die ANSI Norm SLAS 1-2004 Footprint Dimensions. Vorzugsweise wird eine Dimension für die äussere Kante der Nut verwendet, die leicht grösser ist als die Norm, damit auch Platten, welche im oberen Toleranzbereich liegen, verwendet werden können. Mittels dieser Masse wird eine genaue Positionierung der Multiwell-Platte in horizontaler Richtung innerhalb des Beleuchtungsmoduls und somit eine genau Positionierung der einzelnen Wells über den Lichtkanälen gewährleistet. Dies erhöht die Genauigkeit und die Wiederholbarkeit der Experimente, insbesondere da eine gleichmässige und gleichartige Beleuchtung der Wells gewährleistet wird und weil ein Lichteinfall aus anderen Lichtkanälen verhindert wird.

Vorzugsweise weist die Beleuchtungseinheit mehrere lichtemittierende Elemente, vorzugsweise mehrere LED's auf. Die Verwendung von LED's haben sich bewährt. Vorteilhaft ist insbesondere ihre Steuerbarkeit.

Vorzugsweise ist jedem lichtemittierenden Element oder einer Gruppe von lichtemittierenden Elementen genau ein Lichtkanal zugeordnet. Dies gewährleistet die gezielte individuelle Beleuchtung jedes einzelnen Wells.

Die Innenwände der Lichtkanäle sind vorzugsweise lichtundurchlässig ausgebildet, mindestens für die von der Beleuchtungseinheit verwendeten Wellenlängen. Sie sind somit lichttechnisch voneinander getrennt und Streulicht bzw. Kreuzbeleuchtung in andere Kanäle ist innerhalb der Lichtkanäle vermieden.

Die Lichtkanäle weisen vorzugsweise einen Querschnitt auf, der über die gesamte Länge der Kanäle in Form und Grösse gleichbleibt, evtl. mit Ausnahme von Diffusoren. In einigen Ausführungsformen ist er rund. In anderen Ausführungsformen ist er eckig, vorzugsweise mit gerundeten Ecken. Vorzugsweise ist er quadratisch, vorzugsweise mit gerundeten Ecken. Diese Form ermöglicht die sehr präzise Herstellung von Lichtkanälen in grosser Anzahl, insbesondere 384 in der Zahl, geeignet für 384er Multiwell-Platten. Dies wird als eigenständige Erfindung, unabhängig von der Ausbildung der Positionierungseinheit beansprucht.

Die Lichtkanäle, auch Lichtschächte genannt, sind in einigen Ausführungsformen hohl bzw. leer. In bevorzugten Ausführungsformen sind optische Diffusoren vorhanden, damit das Licht aus jedem Lichtkanal gleichmässig verteilt in die entsprechende Well gelangt. Vorzugsweise sind die optischen Diffusoren in den Lichtkanälen angeordnet, vorzugsweise in ihrem ausgangsseitigen Bereich zur Oberfläche bzw. zur Auflagefläche hin. Vorzugsweise ist jeder der optischen Diffusoren einem einzelnen Lichtkanal zugeordnet. Vorzugsweise sind die optischen Diffusoren in den Lichtkanälen angeordnet, z.B. eingesteckt.

Die optischen Diffusoren sind beispielsweise aus Acrylglas (Plexiglas) oder Quarzglas.

Vorzugsweise ist ein Diffusorträger vorhanden, in welchem die Diffusoren angeordnet sind. In einigen Ausführungsbeispielen ist der Diffusorträger weich bzw. flexibel ausgebildet. In einigen Ausführungsbeispielen ist der Diffusorträger plattenförmig ausgebildet, in anderen Ausführungsformen blockförmig. In weiteren Ausführungsformen sind die Diffusoren in den Durchgangsöffnungen des Trägerblocks der Positionierungseinheit angeordnet.

Vorzugsweise ist zwischen der Beleuchtungseinheit und der Oberfläche der Positioniereinheit mindestens eine weiche bzw. flexible Zwischenlage vorhanden. Sie weist Durchgangsöffnungen auf, die einen Abschnitt der Lichtkanäle bilden. Die mindestens eine flexible Zwischenlage ist zwischen zwei Komponenten des Beleuchtungsmoduls eingeklemmt gehalten, um eine streulichtlose Verbindung von der Beleuchtungseinheit bis zur Oberfläche der Positionierungseinheit zu schaffen. In einigen Ausführungsformen sind flexible Zwischenlagen mit steifen Platten kombiniert, die ebenfalls Durchgangsöffnungen in derselben Rasterung aufweisen.

In einigen Ausführungsformen bildet der Diffusorträger eine der mindestens einen flexiblen Zwischenlage aus. In einigen Ausführungsformen sind weitere flexible Zwischenlagen vorhanden. In einigen Ausführungsformen ist zwischen die LED's der Beleuchtungseinheit eine flexible bzw. weiche Zwischenlage eingelegt.

In einigen Ausführungsformen wird mindestens ein Teil der Länge der Lichtkanäle durch mehrere übereinander angeordnete Komponenten mit den Lichtkanälen entsprechenden Durchgangsöffnungen gebildet, wobei mindestens jede zweite der Komponenten flexibel bzw. weich ausgebildet ist, um eine lückenlose Verbindung der Komponenten untereinander und mit der Oberfläche der Positionierungseinheit sowie mit der Beleuchtungseinheit zu schaffen, damit kein Streulicht benachbarte Wells beeinflussen kann.

Das Beleuchtungsmodul weist vorzugsweise ein Basisteil und einen Deckel auf, die gemeinsam das Gehäuse ausbilden. Vorzugsweise ist das Beleuchtungsmodul stapelbar mit gleich ausgebildeten Beleuchtungsmodulen ausgebildet. Vorzugsweise ist es mindestens mit Deckel und im geschlossenen Zustand stapelbar. Dies ermöglicht eine platzsparende Anordnung und Lagerung.

In bevorzugten Ausführungsformen weist das Beleuchtungsmodul Aussenmasse auf, die der Norm ANSI SLAS 1-2004 Footprint Dimensions entsprechen und in der Länge 127.76 mm ± 0.25 mm und in der Breite 85.48 mm ± 0.25 mm betragen. Zudem ist vorzugsweise ein Flansch vorhanden, der dem Flansch einer Multiwell-Platte gleicht und somit kompatibel mit der Multiwell-Platte ist. Alternativ kann dieser Flansch in den entsprechenden Dimensionen auch vollständig oder teilweise als Ausstülpung am Boden des Beleuchtungsmoduls angeordnet sein. Beispielsweise ist diese Ausstülpung durch nach unten gerichtete Rippen am Boden des Beleuchtungsmoduls ausgebildet.

Entspricht der Flansch bzw. die Rippen den normierten Aussenmassen von Multiwell-Platten, lässt sich anstelle der einzelnen Multiwell-Platten das gesamte Beleuchtungsmodul in Labor- und Bearbeitungsvorrichtungen mit normierten Aufnahmen für Multiwell-Platten einlegen. So lässt sich das gesamte Beleuchtungsmodul mit eingelegter Multiwell-Platte mit Labor-Schüttlern, Robotern und anderen automatisierten Labormanipulatoren betätigen. Es lässt sich beispielsweise in Pipettieranlagen einlegen. Dies ermöglicht eine einfache und effiziente Verwendung in der industriellen Anwendung. Dies wird als eigenständige Erfindung, unabhängig von der Ausbildung der Positionierungseinheit oder der Wellform beansprucht.

Nach unten vorstehende Rippen, Stifte oder andere Anschlagsmittel ermöglichen auch eine Stapelung von mehreren Beleuchtungsmodulen. Dies erleichtert ebenfalls die industrielle Handhabung sowie ermöglicht eine platzsparende Aufbewahrung.

Sind die Anschlagsmittel derart angeordnet und weist das Gehäuse des Behältermoduls, entweder der Deckel oder das Basisteil, Masse zur annähernd verschiebungsfreien Aufnahme einer normierten Multiwell-Platte auf, so dienen diese Anschlagmittel einerseits zur sicheren Stapelung mit Deckel und gleichzeitig als Positionierhilfen für Pipettieranlagen und Ähnlichen, wie auch zur Handhabung mittels Roboter. Die Stapelbarkeit, insbesondere die Stapelbarkeit in Kombination mit der Fähigkeit zur industriellen Handhabung wird hier ebenfalls als separate Erfindung beansprucht.

Es stellt sich somit die Aufgabe der Erfindung, ein einfach handhabbares Beleuchtungsmodul zu schaffen. Diese Aufgabe löst ein Beleuchtungsmodul mit den Merkmalen des Patentanspruchs 13 bzw. 14.

Die Verwendung mindestens einer weichen bzw. flexiblen Zwischenlage wird als separate Erfindung beansprucht.

Es ist eine Aufgabe dieser Erfindung, eine möglichst lichtdichte Verbindung zwischen der Beleuchtungseinheit und dem Wellboden einer Multiwell-Platte zu schaffen. Diese Aufgabe wird durch eine Beleuchtungseinheit mit den Merkmalen des Patentanspruchs 15 gelöst.

In einer Ausführungsform der oben beschriebenen Erfindungen bildet das Beleuchtungsmodul selber ein Beleuchtungsgerät aus. D.h. die zur Betreibung des Moduls notwendigen elektronischen Bauteile wie beispielsweise eine Steuereinheit zur Ansteuerung der einzelnen Lichtquellen sind im Gehäuse angeordnet.

In bevorzugten Ausführungsformen ist eine Beleuchtungsvorrichtung vorhanden mit mindestens einem Beleuchtungsmodul. Die Beleuchtungsvorrichtung weist eine Steuereinheit auf zur Regelung der Lichtintensität der lichtemittierenden Elemente, wobei die lichtemittierenden Elemente vorzugsweise mindestens eines von Folgendem sind: einzeln ansteuerbar und/oder in Gruppen ansteuerbar.

In bevorzugten Ausführungsformen ist eine Beleuchtungsvorrichtung vorhanden, die das Beleuchtungsmodul und eine Steuereinheit sowie vorzugsweise eine Datenverarbeitungseinheit aufweist. Zwischenformen sind auch möglich, d.h. eine Basissteuerung kann im Beleuchtungsmodul vorhanden sein, die mit einer externen Steuerung ergänzt wird. Die externe Steuerung kann ein Steuerungsprogramm sein, das auch einem Computer ausgeführt wird. Dasselbe gilt für die Datenverarbeitungseinheit.

Ist eine zumindest teilweise externe Steuereinheit bzw. Datenverarbeitungseinheit vorhanden, so können mehrere Beleuchtungsmodule mit denselben Einheiten gesteuert bzw. bearbeitet werden. Dies erhöht die Effizienz. Dies ermöglicht auch, mehrere Multiwell-Platten koordiniert zu beleuchten und die einheitliche Produktion oder Experimente auszudehnen.

In einigen Ausführungsformen weist das Beleuchtungsmodul und/oder die Beleuchtungsvorrichtung eine Netzwerkintegration auf, um eine Fernsteuerung und Überwachung, insbesondere mittels externer Geräte, zu ermöglichen.

Die Multiwell-Platte ist üblicherweise ein handelsübliches Produkt. Vorzugsweise ist die innere Oberfläche des Wellbodens flach ausgebildet. In einigen Ausführungsformen weisen die Wells einen runden Querschnitt auf. In einigen Ausführungsformen weisen die Wells einen flachen, d.h. horizontal verlaufenden Wellboden auf. In einigen Ausführungsformen weisen die Wells einen rechteckigen Querschnitt auf. Diese rechteckigen Wells weisen den Vorteil auf, dass eine höhere Zahl von Wells innerhalb derselben Grösse einer Platte möglich ist. Die Ecken dieser Wells sind vorzugsweise abgerundet. Beleuchtungsvorrichtungen mit eckigen, insbesondere quadratischen oder rechteckigen Durchgangsöffnungen werden hiermit als eigenständige Erfindung beansprucht, ohne die kennzeichnenden Merkmale der unabhängigen Patentansprüche.

Weitere Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Figur 1: eine perspektivische Explosionsdarstellung eines erfindungsgemässen Beleuchtungsmoduls in einer ersten Ausführungsform;
- Figur 2: eine perspektivische Darstellung des Beleuchtungsmoduls gemäss Figur 1;
- Figur 3: eine perspektivische Explosionsdarstellung des Beleuchtungsmoduls gemäss Figur 1 von unten;
- Figur 4: eine perspektivische Darstellung des Beleuchtungsmoduls gemäss Figur 1 von unten;
- Figur 5: einen Teilschnitt durch das Beleuchtungsmodul gemäss Figur 1 ohne Deckel;
- Figur 6: einen Teilschnitt durch ein Basisteil des Beleuchtungsmoduls gemäss Figur 1 ;
- Figur 7: eine Explosionsdarstellung des Beleuchtungsmoduls gemäss Figur 1;
- Figur 8: einen Querschnitt durch das Beleuchtungsmodul gemäss Figur 1 ohne Deckel;
- Figur 9: einen Querschnitt durch das Basisteil des Beleuchtungsmoduls gemäss Figur 1;
- Figur 10: eine Seitenansicht des Beleuchtungsmoduls gemäss Figur 1 ohne Deckel und ohne Multiwell-Platte;
- Figur 11: eine Seitenansicht des Beleuchtungsmoduls gemäss Figur 1 ohne Deckel, jedoch mit Multiwell-Platte;
- Figur 12: einen Teilschnitt durch das Beleuchtungsmodul gemäss Figur 11;
- Figur 13: einen Ausschnitt X des Teilschnitts gemäss Figur 12 in vergrösserter Darstellung;
- Figur 14: eine Ansicht des Beleuchtungsmoduls gemäss Figur 1 von oben ohne Deckel, mit aufgesetzter Multiwell-Platte;
- Figur 15: eine Ansicht des Beleuchtungsmoduls gemäss Figur 14 von oben ohne Deckel und ohne Multiwell-Platte;
- Figur 16: eine Ansicht einer Variante des erfindungsgemässen Beleuchtungsmoduls gemäss Figur 1 von oben für 6 Wells;
- Figur 17: eine Ansicht einer Variante des erfindungsgemässen Beleuchtungsmoduls gemäss Figur 1 von oben für 24 Wells;
- Figur 18: eine Ansicht einer Variante des erfindungsgemässen Beleuchtungsmoduls gemäss Figur 1 von oben für 96 Wells;
- Figur 19: einen Ausschnitt Z des Beleuchtungsmoduls gemäss Figur 18 in vergrösserter Darstellung;
- Figur 20: eine Ansicht einer Variante des erfindungsgemässen Beleuchtungsmoduls gemäss Figur 1 von oben für 384 Wells;
- Figur 21: einen Ausschnitt Y des Beleuchtungsmoduls gemäss Figur 20 in vergrösserter Darstellung
- Figur 22: eine perspektivische Explosionsdarstellung eines erfindungsgemässen Beleuchtungsmoduls in einer zweiten Ausführungsform;
- Figur 23: eine perspektivische Darstellung des Beleuchtungsmoduls gemäss Figur 22;
- Figur 24: einen Teilschnitt durch ein Basisteil des Beleuchtungsmoduls gemäss Figur 22;
- Figur 25: einen Querschnitt durch das Basisteil des Beleuchtungsmoduls gemäss Figur 22;
- Figur 26: eine Explosionsdarstellung des Beleuchtungsmoduls gemäss Figur 22;
- Figur 27: eine perspektivische Explosionsdarstellung eines erfindungsgemässen Beleuchtungsmoduls in einer dritten Ausführungsform;
- Figur 28: eine perspektivische Darstellung des Beleuchtungsmoduls gemäss Figur 27;
- Figur 29: eine perspektivische Explosionsdarstellung Beleuchtungsmoduls gemäss Figur 27 mit anderen Einlegeplatten;
- Figur 30: eine perspektivische Darstellung des Beleuchtungsmoduls gemäss Figur 29;
- Figur 31: einen Querschnitt durch das Beleuchtungsmodul gemäss Figur 27;
- Figur 32: einen Ausschnitt des Querschnitts gemäss Figur 31 in vergrösserter Darstellung;
- Figur 33: einen Ausschnitt eines Querschnitts des Beleuchtungsmoduls gemäss Figur 29.

Gleiche Teile sind mit gleichen Bezugszeichen versehen.

### BESCHREIBUNG BEVORZUGTER AUSFÜHRUNGSFORMEN

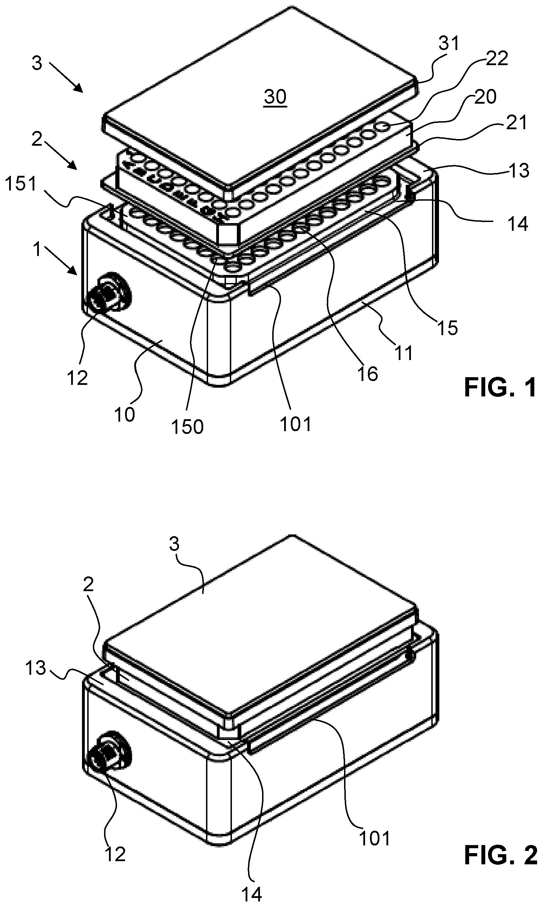

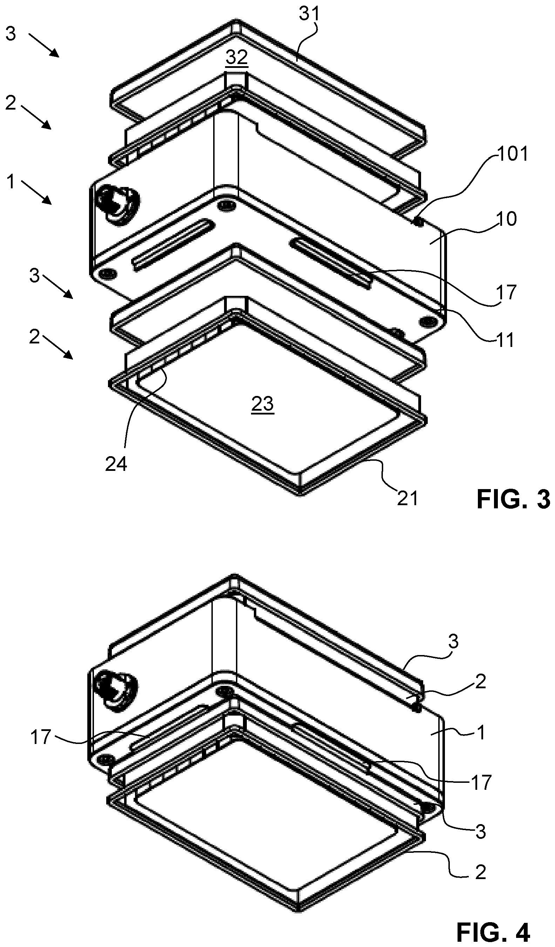

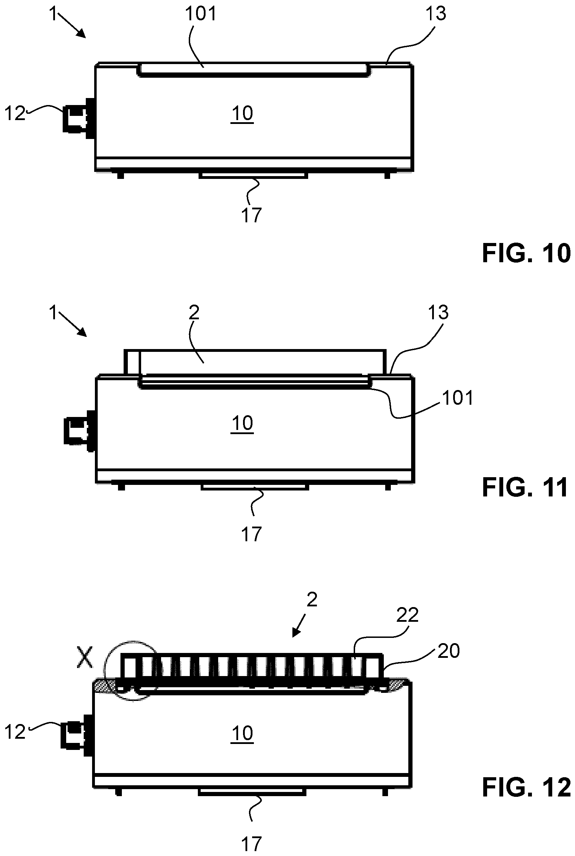

In den Figuren 1 bis 15 ist ein erstes Ausführungsbeispiel eines erfindungsgemässen Beleuchtungsmoduls dargestellt.

Es weist ein Basisteil 1 zur Aufnahme einer Multiwell-Platte 2 und einen Deckel 3 zur Überdeckung der Multiwell-Platte 2 auf.

Das Basisteil 1 weist einen Grundkörper 10 und einen Boden 11 auf.

Der Grundkörper 10 ist vorzugsweise monolithisch, d.h. einteilig, ausgebildet. Er umschliesst eine Kammer 103 und ist nach unten offen ausgebildet. Nach oben ist der Grundkörper 10 mit Ausnahme von Durchgangsöffnungen 150 geschlossen ausgebildet. Die obere Seite des Grundkörpers 10 bildet einen umlaufenden äusseren Rand 13, eine daran anschliessende vertiefte umlaufende Nut 14 und einen bezüglich der Nut 14 erhöhten Trägerblock 15 auf. Die Durchgangsöffnungen 150 sind Teil des Trägerblock 15. Sie führen in die Kammer 103 und sind Teil von weiter unten im Text beschriebenen Lichtkanäle 16. Die geschlossenen Bereiche der Oberfläche des Trägerblocks 15 bilden vorzugsweise eine plane, d.h. ebene, Oberfläche 151 aus. Diese Oberfläche 151 liegt tiefer als die Oberfläche des äusseren Randes 13 oder fluchtet mit dieser. In anderen Ausführungsformen überragt diese Oberfläche 151 den äusseren Rand 13. Die Oberfläche 151 dient als Auflagefläche für die Multiwell-Platte 2, genauer für ihren weiter unten beschriebenen Wellboden 23.

Der Trägerblock 15 ist vorzugsweise einteilig mit dem Grundkörper 10 ausgebildet. In anderen Ausführungsformen ist er ein auswechselbarer Einsatz.

Die Durchgangsöffnungen 150 entsprechen in Anzahl, Grösse und Abstand den Wells 22 einer mit diesem Beleuchtungsmodul zu verwendenden Multiwell-Platte 2. D.h. gemäss den aktuellen und geplanten Produkten sind 6, 24, 96 oder 384 Lichtkanäle vorhanden. Die Durchgangsöffnungen 150 erstrecken sich von einer unteren Oberfläche des Trägerblocks 15 bis zu seiner obersten Oberfläche 151 und verlaufen senkrecht zur Oberfläche 150.

Der Trägerblock 15, insbesondere die Wände der Durchgangsöffnungen 150, sind lichtundurchlässig ausgebildet, mindestens für die in der Beleuchtungseinheit 5 verwendeten Wellenlängen.

Mindestens ein Stecker 12 ist am Grundkörper 10 befestigt und ragt von aussen in die Kammer 103 hinein. Er dient als Anschluss für Strom und Datenkabel.

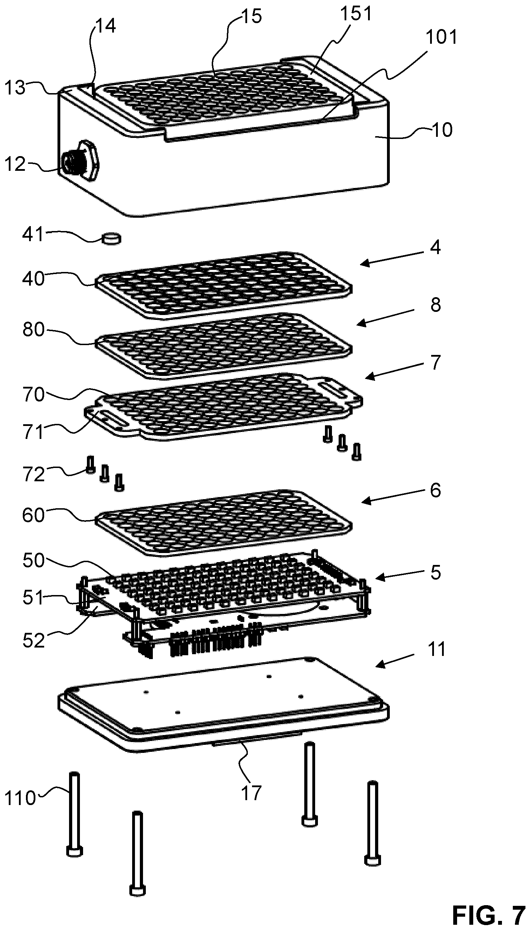

Der Boden 11 ist am Grundkörper 10 befestigt, vorzugsweise ist er lösbar befestigt. Die hierzu verwendeten Schrauben 110 sind in Figur 7 erkennbar. Der Boden 11 verschliesst die Kammer 103 des Grundkörpers 10.

Das Basisteil 1 ist vorzugsweise aus Aluminium und der Deckel 3 ist vorzugsweise aus Kunststoff gefertigt.

Der Deckel 3 weist vorzugsweise eine plane Deckfläche 30, eine plane Unterseite 32 und eine umlaufende Seitenwand 31 auf. Er liegt auf der eingelegten Multiwell-Platte 2 auf, wobei seine Seitenwand 31 vorzugsweise im Bereich oberhalb der Nut 14 verläuft und nicht in den äusseren Rand 13 des Basisteils 1 ragt. Ist keine Multiwell-Platte 2 eingelegt, liegt der Deckel 3 vorzugsweise auf dem äusserem Rand 13 auf. Alternative Positionen des Deckels sind in dieser und anderen weiteren Ausführungsformen möglich.

Die Masse des Deckels 3 sind so bemessen, dass er die Multiwell-Platte 2 spielfrei umfasst. Die Aussenmasse und Innenmasse der Deckfläche 30 entsprechen der Deckfläche eines Deckels einer Multiwell-Platte.

Wie in den Figuren 1, 5, und 14 gut erkennbar ist, weist die Multiwell-Platte 2 üblicherweise eine Beschriftung der Zeilen und Spalten auf.

Das Basisteil 1, der Trägerblock 15 und der Deckel 3 weisen eine rechteckige Grundform auf. Das Basisteil 1 und der Trägerblock 15 sind vorzugsweise im Wesentlichen quaderförmig ausgebildet.

Ist eine Multiwell-Platte 2 in das Beleuchtungsmodul eingelegt, so überragt sie üblicherweise den oberen äusseren Rand 13 des Basisteils 1, wie in den Figuren 2, 5 und 11 erkennbar ist.

Um die Entnahme der eingelegten Multiwell-Platte 2 aus dem Basisteil 1 zu erleichtern, weist das Basisteil 1, hier der Grundkörper 10 vorzugsweise an zwei einander gegenüberliegenden Seiten, vorzugsweise an den Längsseiten, eine seitliche Ausnehmung 101 auf. Dies ist in den Figuren 1 bis 4 und 7 gut erkennbar.

Der obere äussere Rand 13 erstreckt sich über die zwei anderen Seiten (hier die kürzeren Seiten) und über einen Bereich der die Ausnehmung 101 aufweisenden Seiten, um so eine x-y Ausrichtung der Multiwell-Platte 2 zu gewährleisten. Es genügt dabei, wenn der äussere Rand sich über die Ecken der Multiwell-Platte erstreckt.

Am Basisteil 1, hier am Boden 11, sind nach unten ragende Rippen 17 angeordnet. Sie diesen einerseits als Standfüsse für das Beleuchtungsmodul, auf welchen das Beleuchtungsmodul auf einen Tisch oder ein Regal gestellt werden kann. Sie können einteilig am Boden 11 angeformt sein oder daran befestigt sein. Alternativ sind die Rippen 17 beispielsweise als ein umlaufender Steg oder als mehrere einzelne Stifte ausgebildet..

Die Rippen 17 sind in einem Abstand angeordnet, die den Positionierungshilfen von Schüttlern und automatischen Pipettieranlagen entsprechen. Sie entsprechend somit den Nuten 24 der Multiwell-Platten 2, wie weiter unten im Text beschrieben ist.

Dank diesen Massen lassen sich die einzelnen Beleuchtungsmodule mittels Roboter in diese Einheiten einlegen. Die Beleuchtungsmodule sind somit geometrisch kompatibel mit Laborgeräten und erlauben dadurch eine automatische und effiziente Handhabung des gesamten Beleuchtungsmoduls im Labor oder in Produktionsanlagen.

Ferner dienen die Rippen 17 als Anschläge, die ein Stapeln von Beleuchtungsmodulen ermöglicht. Die Masse des Deckels 3 sind so bemessen, dass er innerhalb der Rippen 17 angeordnet werden kann und von diesen begrenzt seitlich fixiert ist. Da die Masse des Deckels 3 derart bemessen sind, dass er eine Multiwell-Platte 2 annähernd verschiebungsfrei hält, sind die Multiwell-Platten 2 sicher im Stapel gehalten und werden nicht verschoben, wenn der ganze Stapel transportiert wird. Auch dies ermöglicht die Verwendung von Robotern.

Die Multiwell-Platte 2 ist ein handelsübliches Produkt. Ihre Aussenmasse sind genormt In den Figuren 1, 3, 5 und 13 ist sie gut erkennbar. Sie weist einen rechteckigen Grundkörper 20 auf, der von einem äusseren, umlaufenden Flansch 21 umgeben ist. Vertiefungen (Wells) 22 sind im Grundkörper 20 matrixförmig angeordnet. Die Spalten und Zeilen, in denen die Wells 22 angeordnet sind, sind vorzugsweise beschriftet. Die Beschriftung ist mit dem Bezugszeichen 26 versehen.

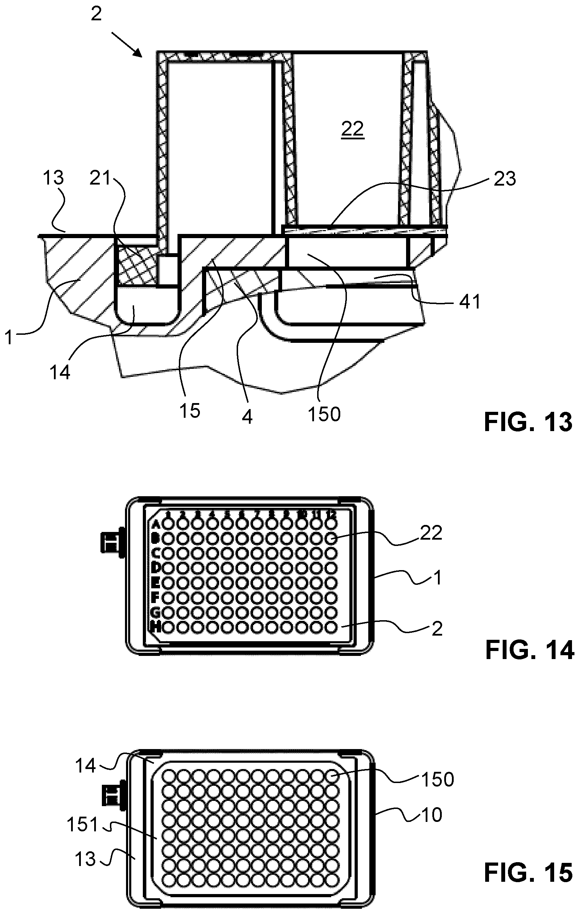

Die Unterseite des Grundkörpers 20 ist mit dem Wellboden 23 verschlossen. Der Wellboden 23 ist plan ausgebildet. Er ist, wie in Figur 13 erkennbar ist, üblicherweise durch eine separate Platte gebildet, die am Grundkörper 20 angeklebt oder angeschweisst ist.

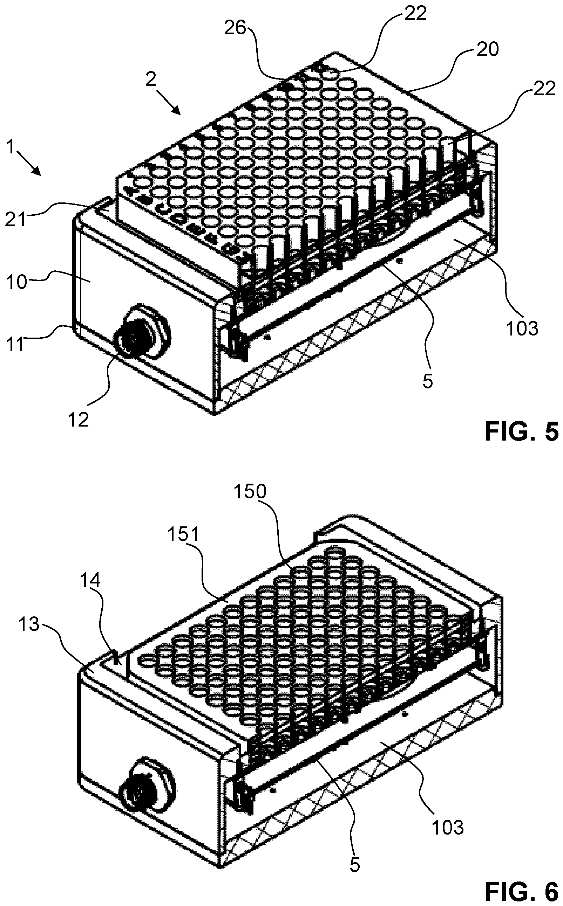

Zwischen Flansch 21 und Wellboden 23 ist eine umlaufende Nut 24 ausgebildet, wie in Figur 5 erkennbar ist. Diese Nut 24 und/oder der Flansch 21 gewährleisten geometrische Kompatibilität mit diversen Laborgeräten, wie Plattenschüttler und Pipettierrobotern. Dies erleichtert die maschinelle Handhabung der einzelnen Multiwell-Platte 2.

Diese Art Multiwell-Platten 2 eignen sich besonders für die Verwendung mit dem Beleuchtungsmodul. Anders ausgebildete Wellplatten können jedoch ebenfalls verwendet werden, sofern die Masse den üblichen Normen entsprechen.

In den Figuren 5 bis 9 sind die weiteren Bestandteile des Beleuchtungsmoduls erkennbar.

In Figur 7 sind die Komponenten erkennbar, die in der Kammer 103 des Basisteils 1 angeordnet sind.

Unterhalb des Trägerblocks 15 ist die Beleuchtungseinheit 5 angeordnet., wobei dazwischen vorzugsweise mindestens eine weitere Komponente angeordnet ist.

Die Beleuchtungseinheit 5 weist eine erste Platine 51 mit lichtemittierenden Elementen 50 und zweite Platine 52 mit Elektronikkomponenten auf, die zur Betreibung und/oder Steuerung der lichtemittierenden Elemente notwendig sind. Diese zwei Platinen 51, 52 sind gemeinsam an einer Innenwand des Grundkörpers 10 fixiert. Vorzugsweise sind sie lösbar befestigt.

Die lösbare Befestigung der Beleuchtungseinheit 5 hat den Vorteil, dass sie ausgewechselt werden kann und das restliche Beleuchtungsmodul auch bei Ausfall oder Alterung der Beleuchtungseinheit 5 länger verwendet werden kann.

Die lichtemittierenden Elemente 50 sind vorzugsweise LED's. Sie sind im Abstand zueinander in Spalten und Zeilen angeordnet und bilden ebenfalls eine Matrix. Sie sind im gleichen Raster angeordnet wie die Durchgangsöffnungen 150 des Trägerblocks 15. Je nach Ausführungsform ist je ein lichtemittierendes Element 50 einer Durchgangsöffnung 150 zugeordnet oder mehrere lichtemittierenden Elemente 50 sind als Gruppe je einer Durchgangsöffnung 150 zugeordnet.

In diesem Beispiel ist ein Diffusorträger 4, auch Diffusorraster genannt, mit optischen Diffusoren 41 sowie mindestens eine Trennplatte 6, mindestens eine Fixierungsplatte 7 und mindestens eine Anpressplatte 8 zwischen der Beleuchtungseinheit 5 und dem Trägerblock 15 vorhanden. Vorzugsweise sind mehrere dieser Platten 4, 6, 7, 8, idealerweise jede zweite, flexibel elastisch ausgebildet, um für einen lichtdichten Übergang zwischen den Platten 4, 6, 7, 8 zu sorgen. Alternativ ist jeweils eine weitere dünne flexible Matte mit analogem Raster zwischen den Platten 4, 6, 7, 8 eingelegt.

Der Diffusorträger 4 sowie die Platten 6, 7, 8 weisen Durchgangsöffnungen 40, 60, 70, 80 in gleichen Raster wie der Trägerblock 15 auf. Die Durchgangsöffnungen 60, 70, 80 der einzelnen Platten 46, 7, 8 und die Durchgangsöffnungen 150 des Trägerblocks 15 haben dieselben Durchmesser bzw. dieselbe Form und Grösse. Die Durchgangsöffnungen 40 des Diffusorträgers 4 sind vorzugsweise leicht grösser. Der Diffusorträger 4 positioniert die Diffusoren 41 passend zu den Durchgangsöffnungen 150.

Die optischen Diffusoren 41 sind zylinderförmige Elemente mit vorzugsweise rundem oder eckigem Querschnitt, die in die einzelnen Öffnungen des Diffusorträgers 4 eingesetzt sind. Sie streuen das Licht des jeweiligen LEDs und gewährleisten eine gleichmässige Beleuchtung des zugeordneten Abschnitts des Wellbodens 23 und somit des zugeordneten Wells 22 der Multiwell-Platte 2. Zudem schützen sie das Innere des Grundkörpers 1 und somit die Beleuchtungseinheit 5 vor einer Verschmutzung durch Staub und andere Partikel. In den Figur 7 ist nur ein optischer Diffusor 41 dargestellt. Es sind jedoch dem Raster entsprechend mehrere Diffusoren 41 vorhanden, für jede Durchgangsöffnung 40 einer.

Die optischen Diffusoren 4 sind beispielsweise aus lichtstreuendem Acrylglas (Plexiglas) oder geschliffenem Quarzglass gefertigt. Der Diffusorträger 4 ist vorzugsweise aus einem flexiblen bzw. elastischen Material gefertigt, insbesondere aus Gummi.

Die Fixierungsplatte 7, auch Kraftraster, genannt, weist Mittel, hier zwei einander gegenüberliegende Flansche 71, zur Befestigung an einer Innenwand des Grundkörpers 10 auf. Sie lässt sich vorzugsweise an den Grundkörper 10 anschrauben. Die Schrauben sind mit dem Bezugszeichen 72 versehen. Die Fixierungsplatte 7 ist vorzugsweise steif ausgebildet. Beispielsweise ist sie aus Aluminium, Chromstahl oder einem anderen Metall gefertigt.

Die Anpressplatte 8, auch Anpressraster genannt, ist gemeinsam mit dem Diffusorträger 4 zwischen der Fixierungsplatte 7 und einer Innenwand des Grundkörpers 10 fixiert. Sie klemmt dank geringfügig kleineren Durchmessern der Durchgangsöffnungen 80 und der Durchgangsöffnungen 150 im Vergleich zu denjenigen des Diffusorträgers 4 die Diffusoren 41 ein. Sie besteht vorzugsweise Aluminium, Kunststoff oder aus Hartgummi. Dank der Anpressplatte 8 wird der Diffusorträger 4 und die darin gehaltenen optischen Diffusoren 41 an die Unterseite des Trägerblocks 15 gepresst.

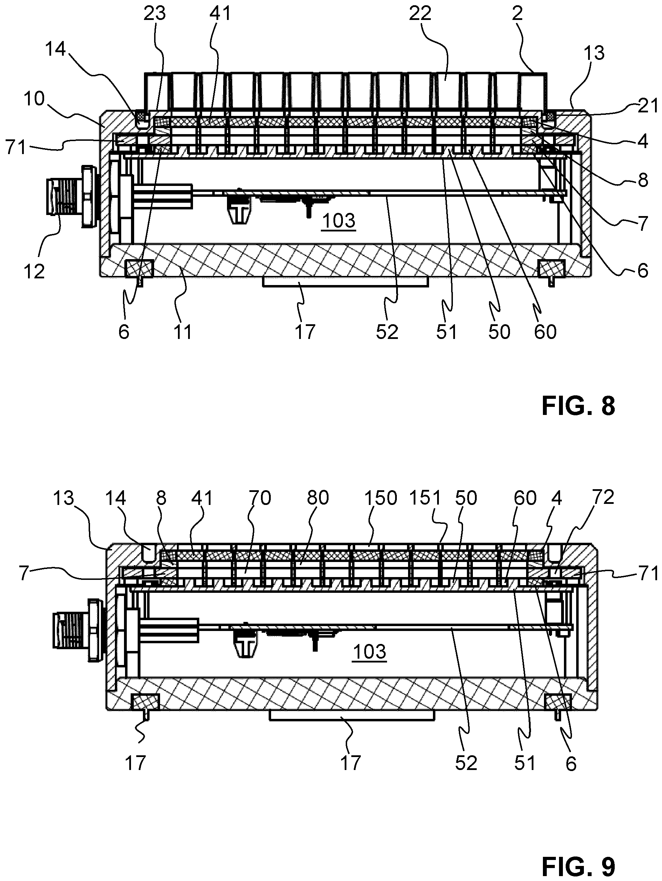

Die mindestens eine Trennplatte 6, auch Pufferraster genannt, ist zwischen der zweiten Zwischenplatte 7 und der Beleuchtungseinheit 5 fixiert. Die einzelnen lichtemittierenden Elemente 50 bzw. die einer Durchgangsöffnung 150 gemeinsam zugeordneten lichtemittierenden Elemente 50 sind dabei in den Durchgangsöffnungen 60 der Trennplatte 6 angeordnet und somit voneinander lichtmässig getrennt. Ihr Licht kann nur durch die senkrecht über ihnen liegende Öffnung nach aussen gelangen. Die Querschnittsfläche der Durchgangsöffnungen 60 der Trennplatte 6 ist vorzugsweise grösser als die Querschnittsfläche des lichtemittierende Elements 50 bzw. der entsprechenden Gruppe von lichtemittierenden Elemente 50. Dies ist in den Figuren 8 und 9 gut erkennbar. Die Trennplatte 6 ist vorzugsweise aus Gummi oder aus einem anderen weichen Material gefertigt.

Vorzugsweise ist genau je eine Platte 6, 7, 8 vorhanden.

Die diversen Durchgangsöffnungen 60, 70, 80, 40 und 150 bilden gemeinsam die Lichtkanäle 16 aus, die von den lichtemittierenden Elementen 50 bis um Wellboden 23 und in die einzelnen Wells 22 führen.

Dank der Ausbildung und gemeinsamen Fixierung der oben beschriebenen einzelnen Komponenten 5, 6, 7, 8, 4, 15 sind Lichtkanäle 16 geschaffen, die kein Streulicht bzw. keine Kreuzbeleuchtung bis zum Wellboden 23 zulassen und dank der sehr schmalen Ausbildung der Wellböden 23 von der Multiwell-Platten 2 somit eine Lichtverschmutzung der einzelnen Wells 22 durch benachbarte Lichtquellen minimieren oder vermeiden. Die Lichtkanäle 16 ermöglichen eine optimale Trennung der einzelnen Lichtquellen und somit eine Abschirmung der einzelnen Wells 22. Durch die elastischen Platten 4, 6, 7, 8 oder durch die Gummimatten zwischen den Platten 4, 6, 7, 8 wird sichergestellt, dass auch der Übergang zwischen diesen Platten 4, 6, 7, 8 lichtdicht ist.

Der modulare Aufbau mit Diffusorträger 4 und den diversen Platten 6, 7, 8 weist den Vorteil auf, dass der Zusammenbau des Beleuchtungsmoduls vereinfacht ist. Diese Komponenten lassen sich am Basisteil 1 anbringen, das Basisteil 1 gelagert werden und da Illuminationsmodul, d.h. die Beleuchtungseinheit 5, kann kurz vor der Auslieferung eingebaut werden. Ferner wird dank dieses Aufbaus kaum Druck auf die empfindliche Beleuchtungseinheit 5 ausgeübt.

Wie in den Figuren 8 und 13 erkennbar ist, liegt die Multiwell-Platte 2 mit ihren Wellboden 23 auf der obersten Oberfläche 151 des Trägerblocks 15 auf. Der umlaufende Flansch 21 der Multiwell-Platte 2, der tiefer liegt als der Wellboden 23, endet jedoch frei innerhalb der Nut 14 und liegt nicht auf.

Dadurch ist gewährleistet, dass der Wellboden 23, d.h. die äussere Oberfläche der Böden der Vertiefungen (Wells) 22 abstandlos auf der Oberfläche 151 des Trägerblocks 15 aufliegt. Dies verhindert ebenfalls eine Lichtverschmutzung durch benachbarte Lichtquellen, da sich kein Spalt zwischen Wellboden 23 und den oberen Öffnungen der Lichtkanäle 16 ausbilden kann. Eine genaue und abstandlose Positionierung der Multiwell-Platte in z-Richtung innerhalb des Beleuchtungsmoduls ist somit gewährleistet.

Wie jedoch ebenfalls in den Figuren 8 und 13 erkennbar ist, steht der Flansch 21 der Multiwell-Platte 2 an der vertikalen Innenwand des äusseren Randes 13 des Basisteils 1 an. Dies ist die äussere Seitenwand der Nut 14. Dadurch ist gewährleistet, dass die Multiwell-Platte auch in horizontaler Richtung, d.h. in den x-y-Richtungen genau positioniert ist. Somit ist gewährleistet, dass die Wells 22 der Multiwell-Platte 2 genau über den jeweiligen Lichtkanälen 16 des Trägerblocks 15 angeordnet sind.

Figur 15 zeigt ein Basisteil 1 mit dem Trägerblock 15 von oben. Figur 14 zeigt das Basisteil 1 mit einer darauf angeordneten Multiwell-Platte 2 mit passendem Lochraster. Es handelt sich um eine 96er Platte.

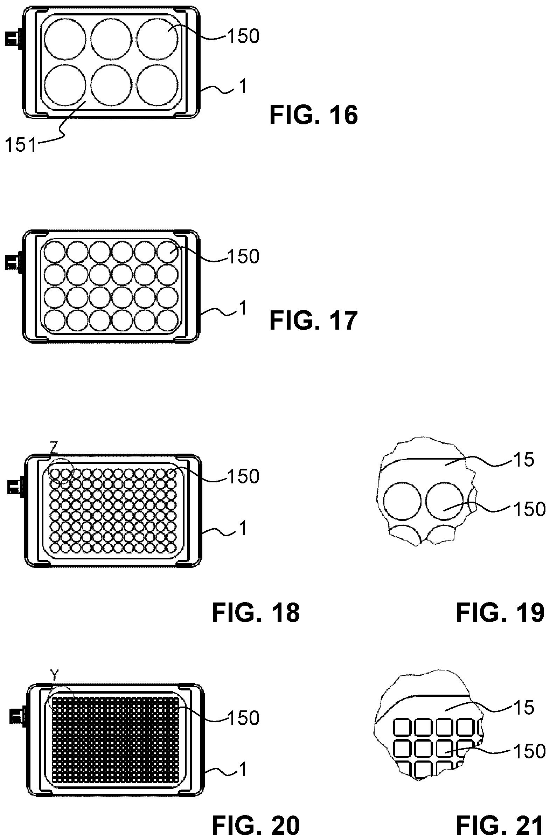

In den Figuren 16 bis 21 sind Beispiele von erfindungsgemässen Beleuchtungsmodulen gezeigt, die entsprechend der mit ihnen zu verwendenden Multiwell-Platten 2 entsprechende Durchgangsöffnungen 150 und somit entsprechende Lichtkanäle 16 aufweisen.

Das Beleuchtungsmodul gemäss Figur 16 ist für 6er Multiwell-Platten 2 geeignet, dasjenige der Figur 17 für 24er Multiwell-Platten 2, dasjenige der Figur 18 für 96er Multiwell-Platten 2. Wie im vergrösserten Abschnitt in Figur 19 erkennbar ist, weisen die Lichtkanäle 16 einen runden Querschnitt auf. Sie weisen über ihre gesamte Länge jeweils, auch bei den bereits oben und den nachfolgend beschriebenen Ausführungsbeispielen, vorzugsweise einen in Form und Grösse gleichbleibenden Querschnitt auf, evtl. mit Ausnahme der Diffusoren 41.

In Figur 20 ist ein Beleuchtungsmodul für 384er Multiwell-Platten 2 gezeigt. Die Lichtkanäle 16 weisen vorzugsweise einen eckigen Querschnitt auf, hier einen quadratischen Querschnitt, wobei vorzugsweise alle Ecken abgerundet sind, wie dies in Figur 21 gut erkennbar ist.

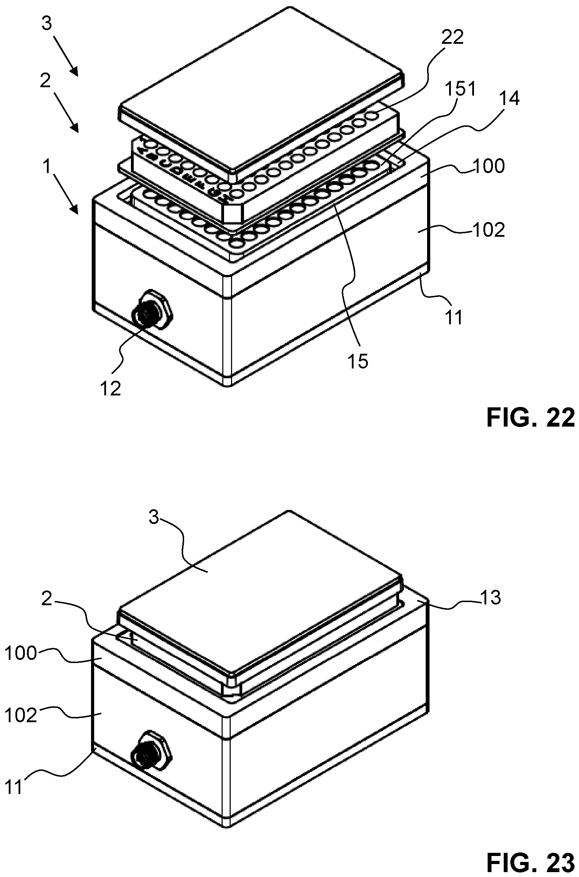

In den Figuren 22 bis 26 ist ein zweites Ausführungsbeispiel eines erfindungsgemässen Behältermoduls gezeigt.

Der Grundkörper des Basisteils 1 ist in diesem Beispiel mehrteilig ausgebildet. Er weist den Boden 11 und ein zweiteiliges Basisteil mit einem oberen Basisteil 100 und einem Rahmenteil 102 auf. Diese Teile sind vorzugsweise aus Aluminium, Kunststoff oder aus einer Kombination davon gefertigt.

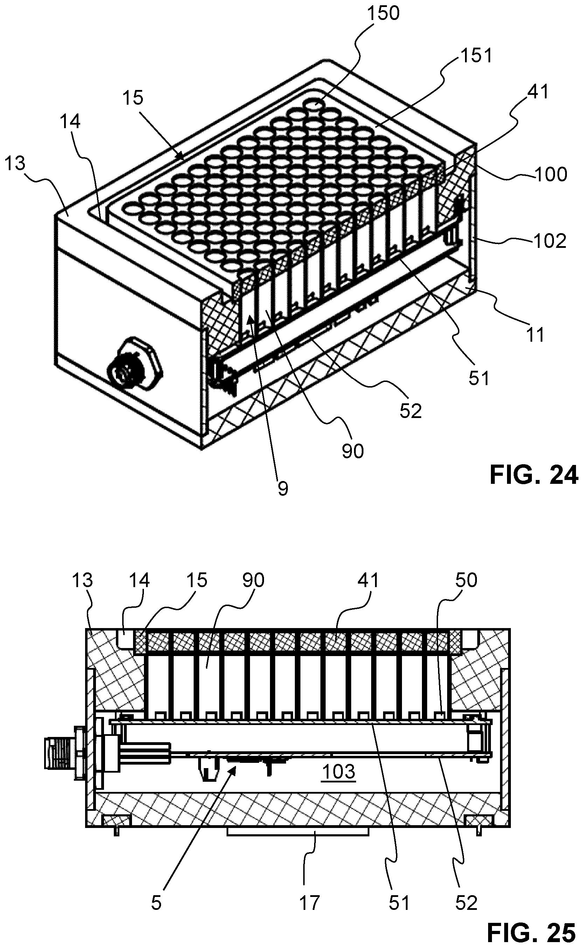

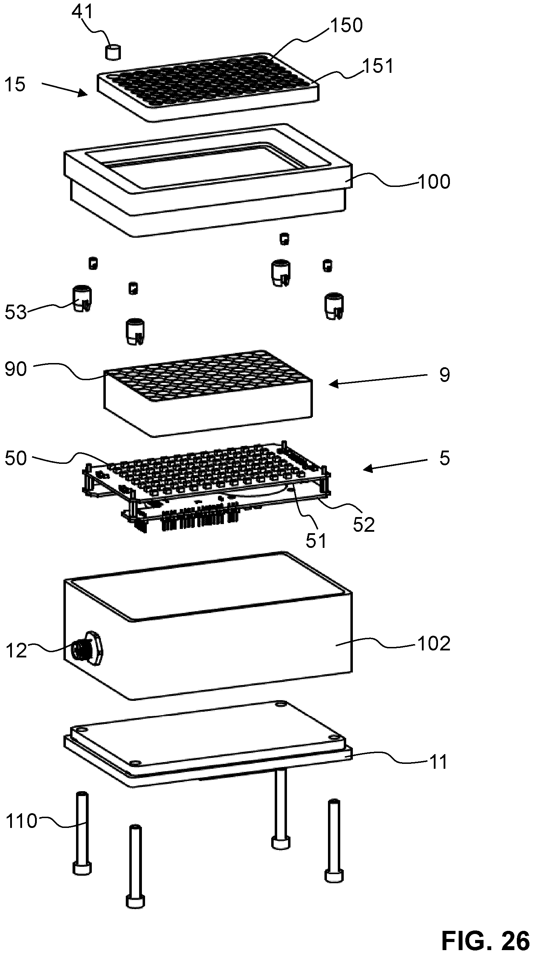

Wie in den Figuren 25 und 26 gut erkennbar ist, ist der Trägerblock 15 mit den Durchgangsöffnungen 150 von oben her in die Nut 14 des oberen Basisteils 100 eingesetzt. Er bildet zugleich den Diffusorträger mit den optischen Diffusoren 41, die in die Durchgangsöffnungen 150 eingelegt sind.

Anstelle der diversen Platten 6, 7, 8 ist ein Lichtkanalblock 9 vorhanden, der zwischen der Beleuchtungseinheit 5 und der inneren Wand des oberen Basisteils 100 angeordnet ist. Der Lichtkanalblock 9 weist Durchgangsöffnungen 90 auf, welche die Abschnitte der Lichtkanäle 16 von den lichtemittierenden Elementen 50, hier den LED's, bis zum Diffusorträger 4 ausbilden. Der Lichtkanalblock 9 trennt dabei die einzelnen _LED's voneinander, indem er auf der Platine aufliegt. Er ist vorzugsweise aus einem Hartgummi gefertigt, der eine genügende Flexibilität aufweist. Dadurch wird ein lichtdichter Übergang zum Trägerblock 15 und zur Beleuchtungseinheit 5 gewährleistet.

Der Lichtkanalblock 9 wird gemeinsam mit der Beleuchtungseinheit 5 an das obere Basisteil 100 angeschraubt. Entsprechende Gewindeeinsätze für Schauben sind in Figur 26 mit dem Bezugszeichen 53 versehen.

Diese Ausführungsform weist den Vorteil auf, dass dasselbe Gehäuse für verschiedene Raster verwendbare ist. Ferner lassen sich einzelne Komponenten, falls defekt oder verschmutzt, einfacher auswechseln, ohne dass das ganze Beleuchtungsmodul ersetzt werden muss.

Im Übrigen trifft bei dieser Ausführungsform die Beschreibung zum ersten Ausführungsbeispiel zu. Insbesondere ist es wiederum mit den Rippen 17 versehen, die eine Stapelbarkeit und eine Positionierung in Laborgeräten ermöglichen. Dies trifft auch auf das nachfolgend beschriebene Ausführungsbeispiel zu.

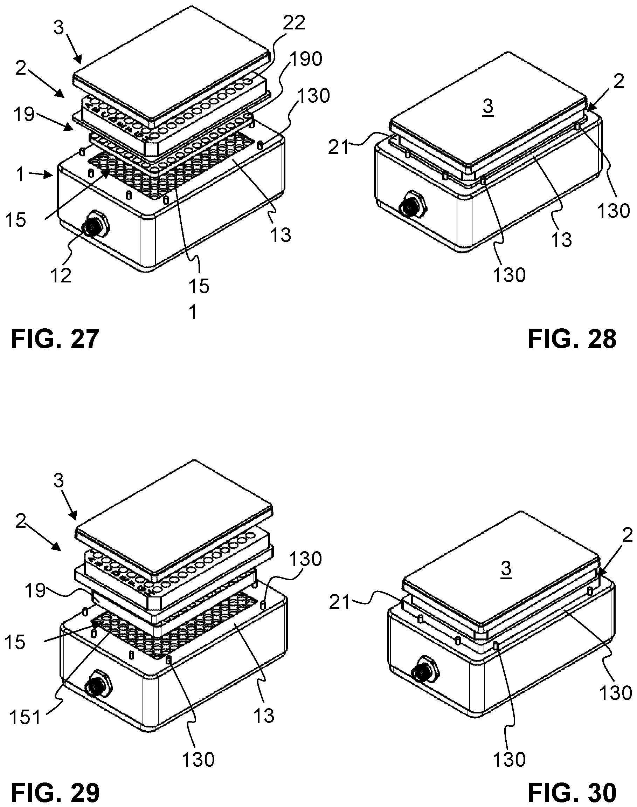

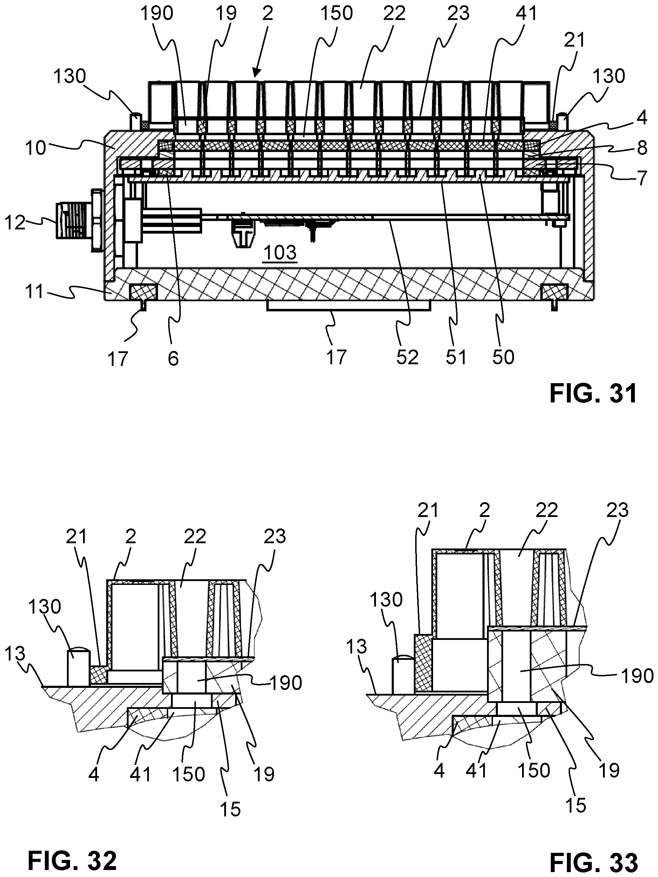

In den Figuren 27 bis 33 ist ein drittes Ausführungsbeispiel beschrieben. Es weist ein Basisteil 1 mit einem einen oder mehrteiligen Grundkörper 10 auf. In diesem Beispiel ist er einteilig ausgebildet. Die in der Kammer 103 angeordnete Komponenten 4, 5, 6, 7, 8 sind dieselben wie im ersten Ausführungsbeispiel.

In dieser Variante ist jedoch keine umlaufende Nut 14 vorhanden. Der äussere Rand erstreckt sich bis zu einer rechteckförmigen Vertiefung, in der die Durchgangsöffnungen 150 angeordnet sind. Der Trägerblock 15 ist in dieser Variante somit nicht eine Erhöhung, sondern eine Vertiefung im Vergleich zum umlaufenden Rand 13 des Basisteils 1. Die Durchgangsöffnungen 150 bilden wiederum ein Raster und die dazwischen ausgebildete Oberfläche 151 bildet eine plane Ebene. Dies ist in den Figuren 27 und 29 gut erkennbar.

Der umlaufende Rand 13 ist mit senkrecht nach oben ragenden Positionierungshilfen, hier mit Stiften 130, versehen. Sie sind über den Umfang verteilt angeordnet und bilden Anschläge für die Multiwell-Plate 2. Die Distanzplatte 190 ist mit Durchgangsöffnungen 190 versehen, die wie die im gleichen Raster und mit gleichem Durchmesser ausgebildet sind wie diejenigen des Trägerblocks 15.

Die Distanzplatte 19 lässt sich in die Vertiefung des oberen Basisteils 100 auf den Trägerblock 15 einlegen. Die Multiwell-Platte 2 wird anschliessend auf die plane Oberfläche 191 dieser Distanzplatte 19 gelegt, wobei die Stifte 130 die Multiwell-Platte 2 genau über die Rasterung positionieren. Die Stifte 130 übernehmen somit die Positionierung der Platte in x-, y-Richtung, welche in den anderen Ausführungsbeispielen durch die Nut 14 erfolgt. Die Durchgangsöffnungen 190 der Distanzplatte 19 verlängern die voneinander getrennten Lichtkanäle 16. Die Positionierung in z-Richtung wird jeweils durch die Auflagefläche definiert, hier die Oberfläche 191 der Distanzplatte 19, in den anderen Ausführungsformen durch die Oberfläche 151 des Trägerblocks 15.

Es sind vorzugsweise zwei oder mehr Distanzplatten 19 mit unterschiedlichen Dicken vorhanden, um entsprechend der Wellbodenhöhe der jeweiligen Multiwell-Platte 2 zur distanzfreien Auflage der Multiwell-Platte 2 auf dem Beleuchtungsmodul zu gewährleisten.

In den Figuren 27, 31 und 32 ist eine dünnere Distanzplatte 19 dargestellt, in den Figuren 29, 30 und 33 eine dickere Distanzplatte 19..

Die einzelnen Bauteile der verschiedenen Ausführungsbeispiele lassen sich gegenseitig auswechseln und zu weiteren Ausführungsbeispielen kombinieren.

Das erfindungsgemässe Beleuchtungsmodul ermöglicht die Verwendung von Multiwell-Platten unterschiedlicher Hersteller und unterschiedlicher Typen und Marken.

### BEZUGSZEICHENLISTE

- 1: Basisteil
- 10: Grundköper
- 100: oberes Basisteil
- 101: seitliche Ausnehmung
- 102: Rahmenteil
- 103: Kammer
- 11: Boden
- 110: Schraube
- 12: Stecker
- 13: äusserer Rand
- 130: Stift
- 14: Nut
- 15: Trägerblock
- 150: Durchgangsöffnung
- 151: Oberfläche
- 16: Lichtkanal
- 17: Rippen
- 19: Distanzplatte
- 190: Durchgangsöffnung
- 191: Oberfläche
- 2: Multiwell-Platte
- 20: Grundkörper
- 21: Flansch
- 22: Vertiefung (Well)
- 23: Wellboden
- 24: Nut
- 26: Beschriftung
- 3: Deckel
- 30: Deckfläche
- 31: Seitenwand
- 32: Unterseite
- 4: Diffusorträger
- 40: Durchgangsöffnung
- 41: optischer Diffusor
- 5: Beleuchtungseinheit
- 50: lichtemittierendes Element
- 51: erste Platine
- 52: zweite Platine
- 53: Gewindeeinsatz
- 6: Trennplatte
- 60: Durchgangsöffnung
- 7: Fixierungsplatte
- 70: Durchgangsöffnung
- 71: Flansch
- 72: Schraube
- 8: Anpressplatte
- 80: Durchgangsöffnung
- 9: Lichtkanalblock
- 90: Durchgangsöffnung

## Patentansprüche

1. Beleuchtungsmodul für eine Multiwell-Platte (2), wobei das Beleuchtungsmodul ein Gehäuse (1, 3), eine Beleuchtungseinheit (5), eine Positionierungseinheit (15, 14, 19, 130) zur Positionierung der Multiwell-Platte (2) im Beleuchtungsmodul und Lichtkanäle (16) aufweist, wobei die Lichtkanäle (16) voneinander getrennte Lichtverbindungen von der Beleuchtungseinheit (5) zu einer Oberfläche (151, 191) der Positioniereinheit (15, 14, 19, 130) schaffen, **dadurch gekennzeichnet, dass** die Positionierungseinheit (15, 14, 19, 130) an dieser Oberfläche (151, 191) eine Auflagefläche ausbildet zur abstandlosen Auflage eines Bodens (23) der Multiwell-Platte (2), der eine Unterseite der zellkulturaufnehmenden Vertiefungen (22) der Multiwell-Platte (2) bildet, wobei die abstandlose Aufnahme unabhängig von einer Wellbodenhöhe, d.h. einer "well bottom elevation", der Multiwell-Platte (2) ist.

2. Beleuchtungsmodul nach Anspruch 1, wobei die Positioniereinheit einen Trägerblock (15) und eine den Trägerblock (15) umlaufende Nut (14) aufweist und wobei eine oberste Oberfläche (151) des Trägerblocks (15) die Auflagefläche bildet.

3. Beleuchtungsmodul nach Anspruch 2,
wobei die Nut (14) eine Tiefe aufweist, die mindestens 3.5 mm beträgt, vorzugsweise mindestens 5 mm,
und/oder
wobei eine äussere Kante der Nut (14) eine Länge von 128.25 +0.2/-0.0 mm und eine Breite von 86 +0.2/-0.0 mm aufweist.

4. Beleuchtungsmodul nach einem der Ansprüche 1 bis 3, wobei die Auflagefläche höhenverstellbar ist.

5. Beleuchtungsmodul nach einem der Ansprüche 1 bis 4, wobei die Oberfläche (191) von einer Distanzplatte (19) mit Durchgangslöchern (190) gebildet ist, die auf einem Basisteil (1) des Gehäuses (1, 3) auswechselbar angeordnet ist und die mittels auf dem Basisteil (1) angebrachten Positionierhilfen (130) positionierbar ist.

6. Beleuchtungsmodul nach einem der Ansprüche 1 bis 5, wobei die Beleuchtungseinheit (5) mehrere lichtemittierende Elemente (50), vorzugsweise mehrere LEDs aufweist.

7. Beleuchtungsmodul nach einem der Ansprüche 1 bis 6, wobei optische Diffusoren (41) vorhanden sind, wobei jeder der optischen Diffusoren (41) einem einzelnen Lichtkanal (16) zugeordnet ist. und wobei die optischen Diffusoren (41) in den Lichtkanälen (16) angeordnet sind.

8. Beleuchtungsmodul nach Anspruch 7, wobei ein Diffusorträger (4) mit Durchgangsöffnungen (40) vorhanden ist, die in einem Raster entsprechend den Lichtkanälen (16) angeordnet sind und in welche die Diffusoren (41) eingelegt sind.

9. Beleuchtungsmodul nach Anspruch 8, wobei der Diffusorträger (4) in einem Basisteil (1) des Gehäuses (1, 3) befestigt ist.

10. Beleuchtungsmodul nach einem der Ansprüche 1 bis 9,
wobei zwischen der Beleuchtungseinheit (5) und der Oberfläche (151, 191) der Positioniereinheit (15, 14, 19, 130) mindestens eine flexible Zwischenlage (4, 6, 7, 8, 9) vorhanden ist,
wobei die flexible Zwischenlage (4, 6, 7, 8, 9) Durchgangsöffnungen (40, 60, 70, 80, 90) aufweist, die einen Abschnitt der Lichtkanäle (16) bilden, und
wobei die mindestens eine flexible Zwischenlage (4, 6, 7, 8, 9) zwischen zwei Komponenten des Beleuchtungsmoduls eingeklemmt gehalten ist, um eine streulichtlose Verbindung von der Beleuchtungseinheit (5) bis zur Oberfläche (151, 191) der Positionierungseinheit (15, 14, 19, 130) zu schaffen.

11. Beleuchtungsmodul nach einem der Ansprüche 1 bis 10, wobei das Beleuchtungsmodul stapelbar mit gleich ausgebildeten Beleuchtungsmodul ausgebildet ist.

12. Beleuchtungsvorrichtung mit mindestens einem Beleuchtungsmodul gemäss einem der Ansprüche 1 bis 11, wobei die Beleuchtungsvorrichtung eine Steuereinheit aufweist zur Regelung der Lichtintensität der lichtemittierenden Elemente, wobei die lichtemittierenden Elemente vorzugsweise mindestens eines von Folgendem sind: einzeln ansteuerbar und/oder in Gruppen ansteuerbar.

13. Beleuchtungsmodul für eine Multiwell-Platte (2), insbesondere ein Beleuchtungsmodul nach einem der Ansprüche 1 bis 12, wobei das Beleuchtungsmodul ein Gehäuse (1, 3), eine Beleuchtungseinheit (5), eine Positionierungseinheit (15, 14, 19, 130) zur Positionierung der Multiwell-Platte (2) im Beleuchtungsmodul und Lichtkanäle (16) aufweist, die voneinander getrennte Lichtverbindungen von der Beleuchtungseinheit (5) zu einer Oberfläche (151, 191) der Positioniereinheit (15, 14, 19, 130) schaffen, **dadurch gekennzeichnet, dass** das Gehäuse (1, 3) Anschlagmittel (17) mit einem Innenmass aufweist, das einem normierten Aussenmass der Multiwell-Platte (2) entspricht, so dass es verschiebungsfrei auf der Multiwell-Platte (2) aufliegt, und so dass das Beleuchtungsmodul mit gleich ausgebildeten weiteren Beleuchtungsmodulen stapelbar ist.

14. Beleuchtungsmodul für eine Multiwell-Platte (2), insbesondere ein Beleuchtungsmodul nach einem der Ansprüche 1 bis 13 wobei das Beleuchtungsmodul ein Gehäuse (1, 3), eine Beleuchtungseinheit (5), und eine Positionierungseinheit (15, 14, 19, 130) zur Positionierung der Multiwell-Platte (2) im Beleuchtungsmodul und Lichtkanäle (16) aufweist, die voneinander getrennte Lichtverbindungen von der Beleuchtungseinheit (5) zu einer Oberfläche (151, 191) der Positioniereinheit (15, 14, 19, 130) schaffen, **dadurch gekennzeichnet, dass** das Gehäuse (1, 3) Anschlagmittel (17) aufweist, die einem normierten Aussenmass (21, 24) der Multiwell-Platte (2) entsprechen, so dass das Beleuchtungsmodul anstelle einer Multiwell-Platte (2) in entsprechend normierte Aufnahme von Vorrichtungen, insbesondere Pipettieranlagen oder Schüttelanlagen, positionsgenau aufnehmbar ist.

15. Beleuchtungsmodul für eine Multiwell-Platte (2), insbesondere ein Beleuchtungsmodul nach einem der Ansprüche 1 bis 14, wobei das Beleuchtungsmodul ein Gehäuse (1, 3), eine Beleuchtungseinheit (5), eine Positionierungseinheit (15, 14, 19, 130) zur Positionierung der Multiwell-Platte (2) im Beleuchtungsmodul und Lichtkanäle (16) aufweist, die voneinander getrennte Lichtverbindungen von der Beleuchtungseinheit (5) zu einer Oberfläche (151, 191) der Positioniereinheit (15, 14, 19, 130) schaffen, **dadurch gekennzeichnet,**
**dass** zwischen der Beleuchtungseinheit (5) und der Oberfläche (151, 191) der Positioniereinheit (15, 14, 19, 130) mindestens eine flexible Zwischenlage (4, 6, 7, 8, 9) vorhanden ist, dass die flexible Zwischenlage (4, 6, 7, 8, 9) Durchgangsöffnungen (40, 60, 70, 80, 90) aufweist, die einen Abschnitt der Lichtkanäle (16) bilden, und dass die mindestens eine flexible Zwischenlage (4, 6, 7, 8, 9) zwischen zwei Komponenten des Beleuchtungsmoduls eingeklemmt gehalten ist, um eine streulichtlose Verbindung von der Beleuchtungseinheit (5) bis zur Oberfläche (151, 191) der Positionierungseinheit (15, 14, 19, 130) zu schaffen.
